# EUROPÄISCHE PATENTSCHRIFT

(11) **EP 2 235 485 B1**
(45) Veröffentlichungstag und Bekanntmachung des Hinweises auf die Patenterteilung: **27.02.2013**
(21) Anmeldenummer: 09704736.9
(22) Anmeldetag: 23.01.2009
(51) Int. Cl.: G01J 5/60, G01N 21/25

(54) **VERFAHREN ZUR NICHTINVASIVEN, OPTISCHEN BESTIMMUNG DER TEMPERATUR VON FLIESSENDEM BLUT IM INNEREN EINES KÖRPERS**
METHOD FOR THE NON-INVASIVE OPTIC DETERMINATION OF THE TEMPERATURE OF FLOWING BLOOD INSIDE A LIVING BODY
PROCÉDÉ DE DÉTERMINATION OPTIQUE NON EFFRACTIVE DE LA TEMPÉRATURE DE SANG CIRCULANT À L'INTÉRIEUR D'UN CORPS VIVANT

(30) Priorität: 25.01.2008 DE 102008006245
(43) Veröffentlichungstag der Anmeldung: 06.10.2010
(73) Patentinhaber: Nirlus Engineering AG, 23560 Lübeck (DE)
(72) Erfinder: HERRMANN, Vera, 23560 Lübeck (DE)
(74) Vertreter: von dem Borne, Andreas
(86) Internationale Anmeldenummer: PCT/EP2009/000440
(87) Internationale Veröffentlichungsnummer: WO 2009/092603

(56) Entgegenhaltungen:
- DE-A1- 10 348 958
- US-A- 5 455 177
- US-A1- 2004 099 815
- Veronica Hollis: "Non-invasive monitoring of brain tissue temperatureby near-infrared spectroscopy" 2003, http://www.medphys.ucl.ac.uk/research/borg /homepages/veronica/veronica_2003.html , XP002541690 Chapter 5

## Beschreibung

Die Erfindung betrifft ein Verfahren zur nichtinvasiven, optischen Bestimmung der Temperatur von Blut gemäβ Anspruch 1 wobei das zu untersuchende Medium mit infrarotem und/oder sichtbaren Licht im Bereich einer Absorptionslinie beleuchtet wird, deren Lage von der Temperatur des Mediums abhängt und wobei die Absorption des Lichtes im Bereich der Absorptionslinie gemessen und aus dieser Messung durch Vergleich mit Kalibrierungsdaten die Temperatur ermittelt wird. - Medium meint im ein wasserhaltiges Medium z.B. lebendes Gewebe und erfindungsgemäβ (fließendes) Blut innerhalb eines menschlichen Körpers. Absorption meint einerseits das z.B. in Transmission gemessene Absorptionsverhalten, aber andererseits auch das von der Absorption abhängige Rückstreuverhalten.

Die Bestimmung der Temperatur, z.B. eines menschlichen Körpers, spielt in der Medizin in unterschiedlichsten Bereichen eine bedeutende Rolle, z.B. bei der Temperaturüberwachung von Intensivpatienten. Dabei wird in der Praxis häufig die nichtinvasive Messung der Körpertemperatur mittels Ohrthermometern angewendet, wobei diese Art der Messung auf die "diskrete" Anwendung, d.h., die Messung in regelmäßigen Zeitabständen beschränkt ist. Für eine kontinuierliche Temperaturüberwachung werden in der Praxis bislang invasive Meßmethoden angewendet, bei denen Sonden oder Katheter mit integrierten Sensoren in den Körper eingeführt oder eingebracht werden.

Außerdem besteht im Zusammenhang mit der nichtinvasiven Messung der Konzentration von Blutbestandteilen und insbesondere im Zusammenhang mit der Messung der Glukosekonzentration in fließendem bzw. pulsierendem Blut das Bedürfnis der Temperaturbestimmung "in situ", da derartige Messungen unter Einsatz von Kalibrierkurven in der Regel von der Temperatur abhängig sind (vgl. DE 10 2006 036 920 und DE 103 11 408 B3). Demnach sind verschiedene optische Verfahren der Nahinfrarot-Spektroskopie (NIRS) bekannt, die auf nichtinvasive Weise anhand von Absorptionsänderungen von Licht im nahinfraroten Wellenlängenbereich die Messung der Konzentration von Blutbestandteilen und beispielsweise die Messung der Glukosekonzentration erlauben. Von Bedeutung ist hier die Tatsache, dass das lebende Gewebe für elektromagnetische Strahlung im roten und im Infrarotbereich weitgehend transparent ist, so dass es möglich ist, innerhalb dieses "biologischen Fensters" in das Gewebe in Tiefen von einigen Millimeter bis zu einigen Zentimeter "hineinzusehen". Mit Hilfe von beispielsweise Ultraschallstrahlung lässt sich das Zielgewebe lokalisieren, so dass gezielt optische Absorptionsmessungen in dem lokalisierten Gewebe in verhältnismäßig großer Tiefe des Körpers durchgeführt werden können (vgl. DE 103 11 408 B3 und DE 10 2006 036 920). Weiterer Stand der Technik ist bekaunt aus US2004099815 A1 und De 10348958A1.

Dabei ist zu beachten, dass im Bereich dieses so genannten biologischen Fensters "diskrete" Wasserabsorptionsbanden liegen, welche bei den oben beschriebenen Messungen der Konzentration von Blutbestandteilen in der Regel gemieden werden. Es ist jedoch bekannt, dass die Lage (und folglich die Wellenlänge) dieser Absorptionsmaxima und auch die Höhe der Absorptionslinie (und folglich die Größe bzw. das Maß der Absorption) von der Temperatur des Mediums, z.B. des Wassers abhängen. Aus diesem Grunde wurde bereits vorgeschlagen, die Temperaturabhängigkeit der Absorption im Bereich dieser Wasser-Absorptionsbanden auszunutzen, um die Temperatur des wasserhaltigen Mediums zu bestimmen. Dazu wurde vorgeschlagen, die Verschiebung der Absorptionslinie spektroskopisch aufzunehmen (vgl. K.H. Norris, Beltsville, MD 20705, USA "Possible medical applications of NIR"). Die insoweit bekannte Methode ist jedoch verhältnismäßig aufwendig, da stets ein vollständiges Spektrum aufgenommen werden muss und folglich ein "Wellenlänge-Scan" gefahren wird. Im Übrigen ist die Linienverschiebung verhältnismäßig gering, so dass mit sehr hohen Spektrometerauflösungen gearbeitet werden muss.

Ein ähnliches Verfahren ist aus der US 2005/0083992 A1 bekannt geworden. Dort wird die Temperatur-Abhängigkeit der Wasser-Absorptionslinie bei einer Wellenlänge von etwa 1450 nm zur Temperaturbestimmung angewendet. Auch bei dem insoweit bekannten Verfahren werden im Großen und Ganzen vollständige Spektren über einen verhältnismäßig großen Wellenlängenbereich aufgenommen, d.h., es erfolgt die vollständige Vermessung der Absorptionslinie und der Vergleich mit entsprechenden Kalibrierungsdaten.

Ausgehend von dem bekannten Stand der Technik liegt der Erfindung die Aufgabe zugrunde ein Verfahren zur nichtinvasiven, optischen Bestimmung der Temperatur von Blut zu schaffen, welches auf einfache und nichtinvasive Weise eine exakte Bestimmung der Temperatur des Bluts ermöglicht. Das Verfahren soll sich zur Messung der Temperatur im Innern eines Körpers, z.B. zur Messung der Temperatur von Gewebe oder fließendem Blut im Innern eines Körpers eignen. Außerdem soll sich das Verfahren in vorteilhafter Weise mit den bekannten Verfahren zur nichtinvasiven Bestimmung der Konzentration von Blutbestandteilen, z.B. der Messung der Glukosekonzentration in pulsierendem Blut, kombinieren lassen.

Zur Lösung dieser Aufgabe lehrt die Erfindung bei einem gattungsgemäßen Verfahren zur nichtinvasiven, optischen Bestimmung der Temperatur eines Mediums der eingangs beschriebenen Art, dass das Medium mit (zumindest) zwei diskreten Lichtwellenlängen beleuchtet wird, welche im Bereich der Absorptionslinie auf unterschiedlichen Seiten des Absorptionsmaximums liegen, dass aus dem Verhältnis dieser beiden ermittelten Absorptionswerte zueinander zumindest ein von der Temperatur abhängiger Messwert bestimmt wird und dass aus diesem Messwert durch Vergleich mit den zuvor aufgenommen Kalibrierungsdaten die Temperatur bestimmt wird. Mit dem "Verhältnis" der beiden ermittelten Absorptionswerte ist eine vorgegebene "Relation" gemeint, welche auf die beiden Messwerte angewendet werden soll. Besonders bevorzugt ist damit die Bildung der Differenz der beiden beidseitig des Maximums liegenden Absorptionswerte gemeint.

Dabei geht die Erfindung zunächst einmal von der (bekannten) Erkenntnis aus, dass sich im Bereich des biologischen Fensters mehrere Absorptionslinien des Wassers befinden, deren Höhe und insbesondere auch Lage (bzw. Wellenlänge) empfindlich von der Temperatur des wasserhaltigen Mediums abhängt. Es ist nun im Rahmen der Erfindung jedoch nicht erforderlich, die Absorptionslinie vollständig zu vermessen und oder die Lage des Absorptionsmaximums exakt festzustellen. Vielmehr wird im Rahmen der Erfindung auf einfache Weise eine Messung mit zumindest zwei und vorzugsweise lediglich zwei diskreten Lichtwellenlängen durchgeführt, weiche jeweils auf unterschiedlichen Seiten des Absorptionsmaximums liegen. Denn die Erfindung hat erkannt, dass sich bei Änderung der Temperatur durch die Verschiebung des Maximums die Absorptionswerte beidseitig des Maximums in sehr unterschiedlicher Weise empfindlich ändern, so dass - wenn beispielsweise die Differenz dieser beiden Werte ermittelt wird - diese Differenz besonders empfindlich von der Temperatur des Mediums abhängt. Mit anderen Worten kann im Zuge der Auswertung durch die beiden Absorptionspunkte der beiden fest vorgegebenen Wellenlängen eine Gerade hindurchgelegt werden und als Messwert wird z.B. die Steigung dieser Geraden ermittelt, in welche die Differenz dieser beiden Absorptionswerte einfließt. Die Steigung dieser Geraden und insbesondere auch das Vorzeichen dieser Steigung hängen nun ganz empfindlich von der Temperatur ab, so dass eine exakte Temperaturbestimmung auch ohne exakte Bestimmung der Maximum-Verschiebung möglich wird. Es ist lediglich erforderlich, zwei Absorptionswerte für zwei fest vorgegebene Wellenlängen zu messen und diese in der beschriebenen Weise auszuwerten. Dieses wird im Folgenden noch in der Figurenbeschreibung weiter verdeutlicht. Es versteht sich, dass im Zuge der Temperaturbestimmung nach bzw. bei Ermittlung der Absorptionswerte und der daraus gewonnenen Messwerte ein Vergleich mit aufgenommenen Kalibrierungsdaten erfolgt. So lassen sich entsprechende Messungen im Labor bei bekannten Temperaturen durchführen und die bekannten Differenzwerte oder Steigungen lassen sich als Kalibrierungsdaten speichern, so dass sie im Zuge der Messung dann automatisch berücksichtigt werden können. Es sei jedoch darauf hingewiesen, dass es sich bei der beschriebenen "Bildung der Differenz" bzw. Bestimmung der Steigung der Verbindungsgeraden um eine bevorzugte Ausführungsform der Auswertung unter Berücksichtigung der beiden beidseitig des Maximums liegenden Absorptionswerte handelt. Die Erfindung umfasst grundsätzlich andere "Relationen", in welche zwei oder auch mehr Messwerte einfließen, die beidseitig des Absorptionsmaximums liegen.

Bevorzugt wird die erfindungsgemäße Messung mit infrarotem und/oder sichtbarem Licht mit einer Wellenlänge zwischen 600 und 2500 nm, vorzugsweise 800 bis 1600 nm durchgeführt. Versuche haben gezeigt, dass die Messung der Temperatur mit Hilfe von Infrarot-Licht im Bereich der WasserAbsorptionsbande um 970 nm zu hervorragenden Ergebnissen führt. In diesem Fall wird dann zumindest eine Wellenlänge zwischen z.B. 950 und 970 nm und zumindest eine Wellenlänge zwischen z.B. 975 und 1000 nm eingesetzt. Es besteht aber auch die Möglichkeit, mit anderen Wasser-Absorptionsbanden innerhalb des biologischen Fensters zu arbeiten, z.B. im Bereich der WasserAbsorptionsbande um 1450 nm. Grundsätzlich kommt jede Absorptionslinie in Betracht, deren Lage (Wellenlänge des Maximums) von der Temperatur abhängt. Der optimale Bereich für die Messung, d.h., die beiden optimal einzusetzenden Wellenlängen, lassen sich in der Praxis experimentell ermitteln. Es sind stets jeweils eine Wellenlänge unterhalb und eine Wellenlänge oberhalb des Absorptionsmaximums zu wählen. Dabei ist darauf zu achten, dass der Abstand vom Maximum hinreichend groß ist, so dass tatsächlich der beobachtete Effekt eintritt, dass sich die Absorptionswerte bei Temperaturveränderung mit entgegengesetzten Vorzeichen verändern, d.h., auf einer Seite des Maximums größer und auf der anderen Seite des Maximums kleiner werden. Bei einer Temperaturerhöhung soll die Absorption bei einer der Wellenlänge stets wachsen und bei der anderen Wellenlänge stets sinken. Bei einer Temperaturemiedrigung soll sich das gegenteilige Verhalten zeigen. Allerdings darf der Abstand der ausgewählten Wellenlänge nicht zu weit vom Absorptionsmaximum entfernt sein, da dort die Gefahr der Überlagerung mit anderen Linien bzw. Effekten besteht. Es hat sich als zweckmäßig erwiesen, zunächst einen bestimmten Temperaturbereich, z.B. 30°C bis 43°C festzulegen und dann eine mittlere (typische) Temperatur (z.B. 37°C) festzulegen und dort die Wellenlänge des Absorptionsmaximums zu bestimmen. Die ausgewählten Wellenlängen λ₁, λ₂ für die Messung sollten dann z.B. etwa 5 bis 30 nm, vorzugsweise 5 bis 15 nm oberhalb bzw. unterhalb dieser Wellenlänger λ₀ liegen. Dieses gilt insbesondere für den Bereich der Absorptionslinie bei 970 nm. Im Falle der Absorptionslinie um 1450 nm kann gegebenenfalls mit größerem Abstand zum Maximum gemessen werden.

Mit dem erfindungsgemäßen Verfahren besteht zunächst einmal die Möglichkeit, die Temperatur von Flüssigkeiten an einem definierten Ort, z.B. im Labor bzw. außerhalb eines Körpers durchzuführen, ohne dass dort weitere störende Effekte auftreten. Von besonderer Bedeutung ist jedoch die Tatsache, dass sich das erfindungsgemäße Verfahren eignet, um die Temperatur an oder in einem lebenden Körper "in situ" zu messen. Insbesondere gelingt die Messung auch in tief liegenden Bereichen, z.B. kann die Temperatur von fließendem Blut in einer Blutbahn in einem Körper gemessen werden. Dazu schlägt die Erfindung vor, den Ort, an welchem die Temperaturmessung stattfinden soll, gezielt durch geeignete Maßnahmen zu markieren. Dieses gelingt mit Hilfe von Ultraschallstrahlung, so wie dieses beispielsweise in der DE 103 11 408 B3 und der DE 10 2006 036 920 beschrieben wird. So lässt sich das zu untersuchende Gewebe bzw. die Blutbahn mit Ultraschallstrahlung "markieren", indem (gepulste) Ultraschallstrahlung auf den Ort bzw. die Blutbahn fokussiert wird. Im Zuge der Messung der Absorption (bzw. Rückstreuung) des Lichtes für die Temperaturmessung werden dann lediglich die Anteile des in den Detektor einfallenden Lichtes berücksichtigt, welche in zeitlicher Beziehung zu der Ultraschallstrahlung stehen, so dass ganz gezielt eine optische Messung und folglich eine Temperaturbestimmung in einem tief liegenden Bereich eines Körpers durchgeführt werden kann.

Um der Tatsache Rechnung zu tragen, dass der z.B. durch Ultraschallschallung markierte Lichtanteil nicht ausschließlich von der Temperatur des beobachteten Ortes abhängt, sondern auch von der Temperaturdifferenz zwischen der Körperobertläche und dem zu beobachtenden Ort, empfiehlt die Erfindung in einer bevorzugten Weiterbildung, dass zunächst eine Messung der Temperatur an der Oberfläche des Köpers erfolgt. Diese Referenzmessung kann ebenfalls auf die erfindungsgemäße Weise erfolgen, wobei auch dort eine Markierung mittels Ultraschallfokus zweckmäßig sein kann. An der Körperoberfläche könnte aber auch eine herkömmliche Referenzmessung vorgenommen werden, z.B. mit einem Temperaturfühler. An der Oberfläche ist die Intensität des von der Oberfläche rückgestreuten Lichtes nur von der Temperatur der Oberfläche abhängig, weil das Licht keine weiteren Zwischenlagen passieren muss. Damit ist zunächst einmal die Temperatur der Oberfläche eindeutig bestimmt. Anschließend kann dann die Messung im Innern des Körpers erfolgen, wobei dann die Temperaturdifferenz bzw. der Temperaturgradient bezogen auf die Temperatur an der Oberfläche des Körpers ermittelt wird. Die Messung im Bereich der Oberfläche des Körpers bildet folglich eine Referenzmessung, um beim anschließenden Messen im Körperinnem die eventuelle Abhängigkeit des Temperaturgradienten zu eliminieren.

Im Übrigen kann es zweckmäßig sein eine Beeinflussung der spektroskopischen Messung an menschlichem Gewebe durch verschiedene Faktoren zu berücksichtigen, z.B. Hautfarbe, Hautfeuchtigkeit, Dicke und Struktur der zwischenliegenden Gewebeabschnitte, Hämatokritwerte (welche bei jedem Menschen variieren können) sowie Fettpegel im Blut, welcher sich stundenweise im Blut verändert. Aus diesem Grunde kann es zweckmäßig sein, zusätzlich zu der beschriebenen Absorptionsmessung und gegebenenfalls der Referenzmessung an der Körperoberfläche eine Korrekturmessung vorzunehmen, mit welcher sich die beschriebenen Effekte und insbesondere verschiedene Streueffekte an den Zwischenlagen eliminieren lassen. Dazu ist es zweckmäßig, Licht mit einer so genannten "isosbestischen Wellenlänge" in den Körper, z.B. in das Gewebe, einzustrahlen und die Absorption bzw. Rückstreuung zu messen. Eine solche isosbestische Wellenlänge zeichnet sich dadurch aus, dass die Absorption bzw. Rückstreuung ausschließlich von verschiedenen Streueffekten in den Zwischenlagen und nicht vom Absorptionsverhalten des Mediums, z.B. Wasser, abhängt. Mit Hilfe einer solchen Messung bei einer isosbestischen Wellenlänge lassen sich folglich die Absorptionsunabhängigen Streueffekte kompensieren bzw. herausfiltern, so dass insgesamt eine besonders exakte Messung auch in tief liegenden Schichten eines Körpers gelingt. Im Falle eines wasserhaltigen Mediums kann beispielsweise eine isosbestische Wellenlänge von etwa 808 nm verwendet werden.

Insgesamt lässt sich mit dem erfindungsgemäßen Verfahren auf einfache Weise die Temperatur eines Mediums besonders exakt (z.B. mit einer Präzision ± 0,01 °C) bestimmen. Es kann die Temperatur an Körperoberflächen oder besonders bevorzugt auch die Temperatur im Innern eines Körpers ermittelt werden, und zwar auf nichtinvasive und optische Weise. Diese Maßnahmen ermöglichen z.B. auch eine exakte Bestimmung der Konzentration von Blutbestandteilen und insbesondere auch der Glukosekonzentration in Blut, da im Zuge einer bekannten Messung der Konzentration (zeitgleich) auch eine nichtinvasive Messung der Temperatur erfolgen kann, und zwar exakt an dem Ort, an dem auch die Konzentration bestimmt wird. Das erfindungsgemäße Verfahren lässt sich jedoch auch in anderen Bereichen vorteilhaft anwenden, z.B. bei der Temperaturüberwachung von Intensivpatienten und bei der Temperaturüberwachung bei der Kryotherapie sowie medizinischen Tumortherapie. Ferner kann eine Temperaturüberwachung bei Neonaten oder auch eine Temperaturüberwachung von Personen im Zuge von Tätigkeiten in thermisch exponierten Umgebungen erfolgen. Weitere Anwendungsfälle sind das Temperaturmonitoring in der Schlafdiagnostik, während der Dialyse oder auch die Temperaturüberwachung von Sportlern. Auch die Temperaturmessung in industriellen Anwendungen, z.B. die Bestimmung der Thermoverteilung in der Bekleidungsindustrie ist ein möglicher Anwendungsfall.

Im Folgenden wird die Erfindung anhand einer lediglich ein Ausführungsbeispiel darstellenden Zeichnung näher erläutert. Es zeigen:
- Fig. 1: einen Versuchsaufbau zur Durchführung eines Verfahrene zur Temperaturbestrimmung
- Fig. 2.: schematisch ein Wasser-Absorptionsspektrum in einem Wellenlängenbereich von etwa 660 nm bis etwa 2400 nm,
- Fig. 3: die Wasserabsorption im Bereich einer Wasserabsorptionsbande um 970 nm bei zwei verschiedenen Temperaturen,
- Fig. 4: Kalibrierungsdaten für eine Messung gemäß Fig. 3, und
- Fig. 5: eine schematische Darstellung des Verfahrens zur Bestimmung der Temperatur im Innern eines Körpers.

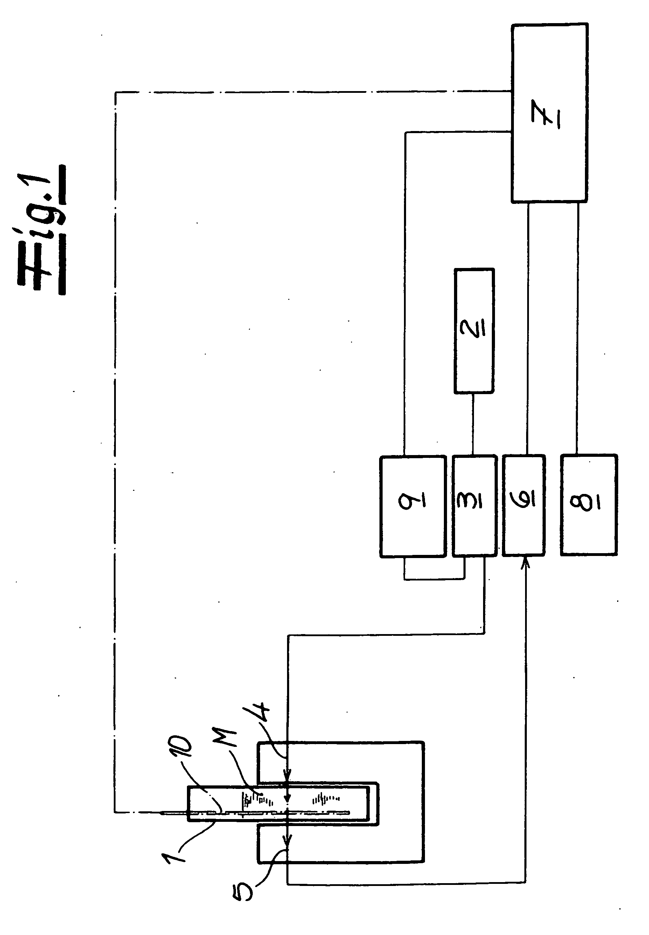

In Fig. 1 ist ein Versuchsaufbau zur Bestimmung der Temperatur T eines wasserhaltigen Mediums auf optischem Wege dargestellt. Mit Hilfe dieses Aufbaus lassen sich optische Absorptionsspektren an einem wasserhaltigen Medium M durchführen. Das wasserhaltige Medium M ist in diesem Laboraufbau in einem Behälter 1 angeordnet. Mit Hilfe eines durchstimmbaren Infrarot-Lasers 2 wird Laserlicht der gewünschten Wellenlänge über einen Koppler 3 und einen Eingangs-Lichtwellenleiter 4 in das Medium M eingestrahlt. Das auf der gegenüberliegenden Seite des Behälters 1 austretende Licht wird über einen Ausgangs-Lichtwellenleiter 5 ausgekoppelt und auf einen Detektor 6 gegeben. Der Detektor 6 ist mit einer Auswerteeinheit 7 verbunden, welche einen Rechner und/oder ein Osziloskop aufweisen kann. In dem Rechner ist der beschriebene Auswertealgorithmus hinterlegt, auf den im Folgenden noch näher eingegangen wird. Außerdem sind in dem Rechner gegebenenfalls zuvor ermittelte Kalibrierungsdaten gespeichert, welche ebenfalls in die Auswertung einfließen können. Auch darauf wird noch eingegangen. An den TriggerEingang des Oszyloskops ist ein TTL-Frequenzgenerator 8 angeschlossen. Ferner ist ein Leistungs-Messgerät 9 vorgesehen, welches ebenfalls mit einerseits dem Rechner 7 und andererseits dem Koppler 3 verbunden ist. Zum Nachweis der Funktionsfähigkeit des erfindungsgemäßen Verfahrens und z.B. auch zur Aufnahme von Kalibrierungsdaten ist in der Fig. 1 ein Thermometer 10 angedeutet, welches die tatsächliche Temperatur des wasserhaltigen Mediums exakt misst, so dass die auf die erfindungsgemäße Weise gewonnenen Temperaturdaten verifiziert werden können. Dabei sei darauf hingewiesen, dass es sich um die schematische Andeutung eines Labor-Aufbaus handelt, welcher in erster Linie dem Nachweis der Funktionsfähigkeit der erfindungsgemäßen Methode dient. In der Praxis erfolgt die optische Bestimmung der Temperatur T in vergleichbarer Weise, indem Laserlicht in den Körper eingestrahlt wird. Es ist dann jedoch zweckmäßig, nicht - wie im Labor - in Transmission zu messen, sondern das rückgestreute Licht zu messen, wobei auch der rückgestreute Anteil das Absorptionsverhalten des Mediums angibt.

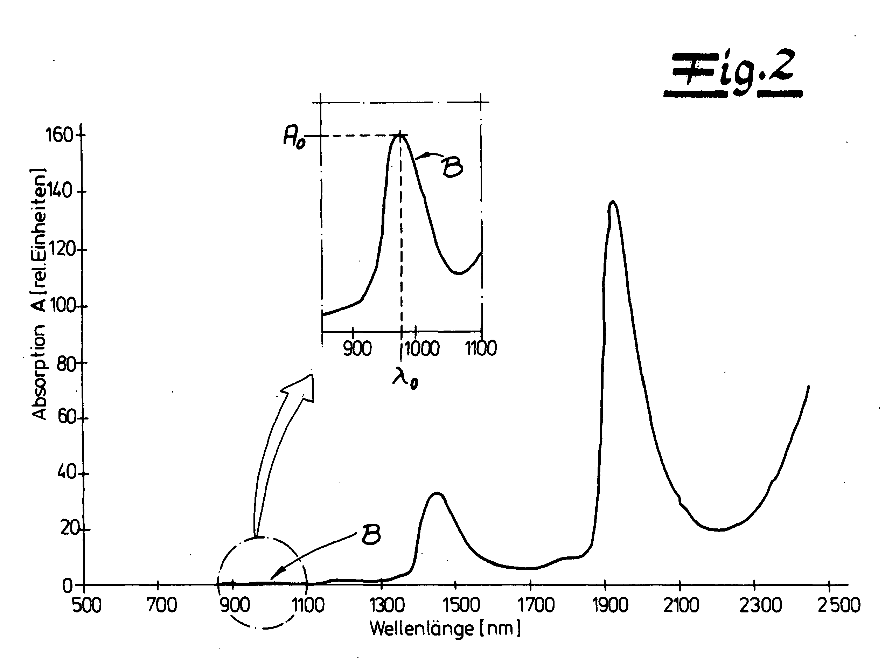

Die physikalischen Zusammenhänge und die Funktionsweise des erfindungsgemäßen Verfahrens sollen nun anhand der Fig. 2 bis 4 erläutert werden.

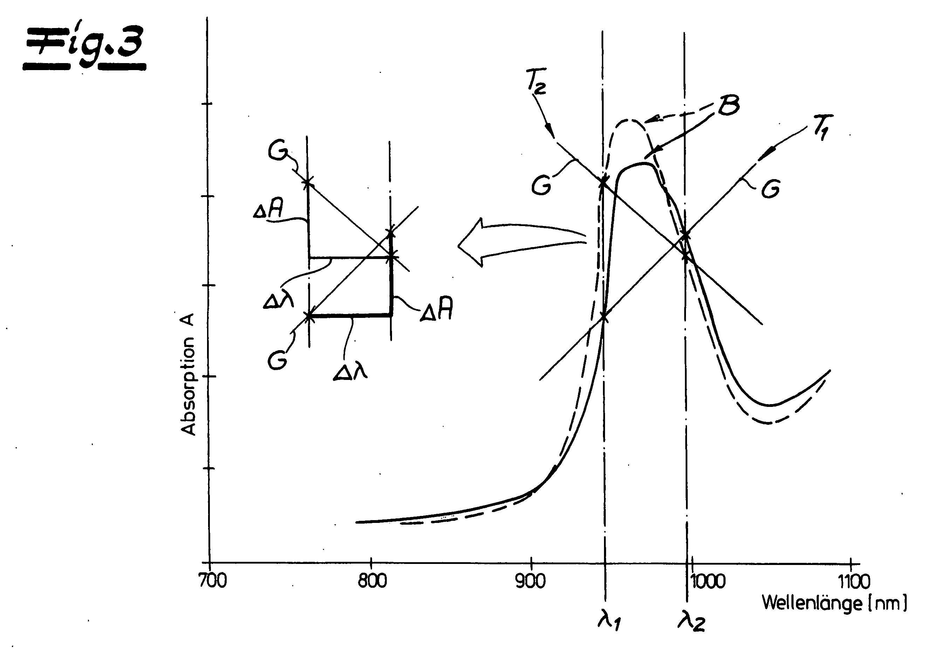

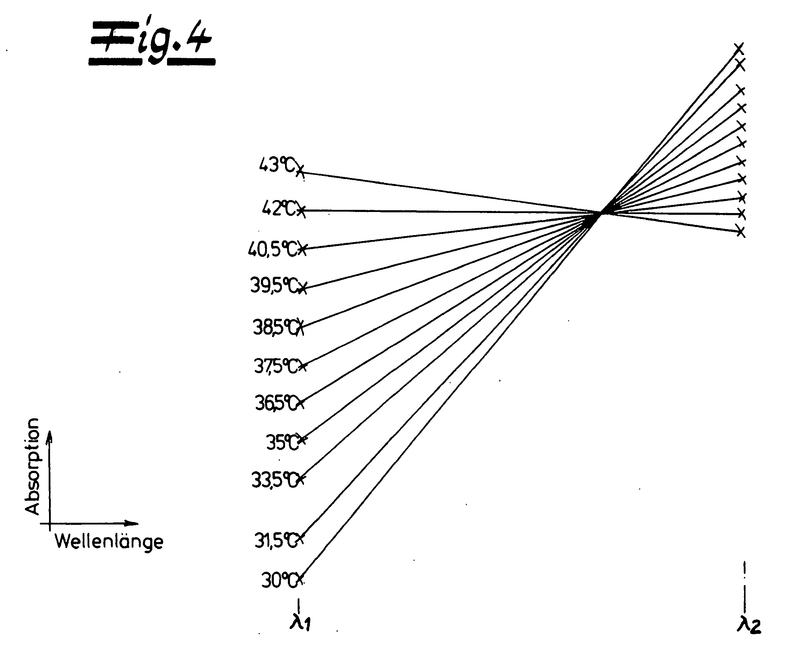

Fig. 2 zeigt zunächst einmal in einer Übersicht beispielhaft und lediglich schematisch ein übliches Absorptionsspektrum von Wasser in einem Wellenlängenbereich von etwa 700 nm bis 2400 nm Es ist bereits die Wasser-Absorptionsbande B im Bereich einer Wellenlänge von λ₀ ≈ 970 nm erkennbar. Wie in der Beschreibung erläutert, hängen sowohl die Lage λ₀ als auch die Höhe A₀ dieser Absorptionslinie B von der Temperatur T des Wassers ab. λ₀ meint folglich die Wellenlänge des Absorptionsmaximums bei einer bestimmten Temperatur, d.h., λ₀ ist temperaturabhängig. Dazu wird beispielhaft auf Fig. 3 verwiesen, welche die Absorption A im Bereich dieser Absorptionslinie B für zwei unterschiedliche Temperaturen T₁ = 33 °C und T₂ = 43 °C zeigt. Es ist erkennbar, dass sich die Absorptionslinie B bei höheren Temperaturen hin zu kurzen Wellenlängen verschiebt. Im Rahmen der Erfindung wird nun die Absorption im Bereich dieser Absorptionslinie B gemessen, und zwar lediglich für zwei fest vorgegebene Wellenlängen λ₁ und λ₂, welche auf unterschiedlichen Seiten des Absorptionsmaximums (A₀, λ₀) liegen. Diese Wellenlängen sind in Fig. 3 ebenfalls eingezeichnet. Dabei ist zu beachten, dass die Lage des Maximums und folglich λ₀ selbst temperaturabhängig ist. Die Wellenlängen λ₁ und λ₂ sind unter Berücksichtigung des gewählten Temperaturbereichs so zu wählen, dass sie für sämtliche Temperaturen dieses Bereiches stets auf unterschiedlichen Seiten des (sich verschiebenden) Maximums liegen. Es ist nun in Fig. 3 erkennbar, dass im Bereich der Wellenlänge λ₁ die Absorption für die höhere Temperatur T₂ deutlich größer ist als für die Temperatur T₁. Auf der anderen Seite des Absorptionsmaximums λ₀ verhält sich das anders. Denn dort ist die Absorption für die höhere Temperatur T₂ geringer als für die Temperatur T₁. Dieser Effekt lässt sich weiter verdeutlichen, indem durch die beiden Messpunkte bei einer Temperatur T eine Gerade G hindurchgelegt wird. Fig. 3 zeigt nun, dass die Steigung ΔA/ Δλ dieser Geraden G ganz empfindlich von der Temperatur T des Mediums abhängt. Dieses gilt gleichermaßen für die Differenz ΔA zwischen den Absorptionswerten bei einer bestimmten Temperatur T₁ bzw T₂ für die beiden Wellenlängen λ₁ und λ₂, denn diese Differenz ΔA = A(λ₁) - A(λ₂) bestimmt die Steigung der eingezeichneten Geraden G. Im Rahmen der Erfindung wird nun folglich bei einer bestimmten Temperatur infrarotes Licht eingestrahlt, und zwar lediglich zwei diskrete Wellenlängen λ₁, λ₂ welche jeweils auf unterschiedlichen Seiten des Absorptionsmaximums λ₀ angeordnet sind. Die gemessenen Absorptionswerte werden in eine Relation gesetzt, z.B. wie im Ausführungsbeispiel voneinander subtrahiert, wobei die gebildete Differenz den ermittelten Messwert bildet, welcher empfindlich von der Temperatur abhängig ist. Dieser Messwert, welcher im Ausführungsbeispiel die Differenz der Absorptionswerte bzw. die Steigung der eingezeichneten Geraden G durch die beiden Messpunkte repräsentiert, wird mit zuvor aufgenommenen Kalibrierungsdaten verglichen. Diese Kalibrierungsdaten sind für eine Vielzahl von Temperaturen in Fig. 4 dargestellt. Dort sind jeweils die Absorptionswerte für verschiedene Temperaturen bei den Wellenlängen λ₁ und λ₂ eingezeichnet. Ferner wurden zur Veranschaulichung ebenfalls Geraden durch die jeweils paarweise zugeordneten Punkte hindurchgelegt. Fig. 4 macht nun besonders deutlich, dass die Differenz der Messwerte und folglich auch die Steigung der Geraden empfindlich von der Temperatur abhängt, da es mit steigender bzw. fallender Temperatur insbesondere auch zu einem Vorzeichenwechsel kommen kann. Es wird folglich bei unbekannter Temperatur die Messung gemäß Fig. 2 bei den beiden Wellenlängen λ₁ und λ₂ durchgeführt und anschließend die Differenz der Absorptionswerte gebildet bzw. die Steigung der extrapolierten Geraden G ermittelt, so lässt sich durch Vergleich mit den Kalibrierungsdaten gemäß Fig. 4 die Temperatur T exakt bestimmen, ohne dass eine Verschiebung des Maximums der Absorptionslinie B gemessen werden muss.

Die Fig. 1 bis 4 verdeutlichen die grundsätzliche Funktionsweise des erfindungsgemäßen Verfahrens und veranschaulichen die Durchführung im Labor. Da es sich um eine optische und nichtinvasive Meßmethode handelt, gelingt in vergleichbarer Weise auch die Messung der Temperatur innerhalb eines Körpers, z.B. die Bestimmung der Temperatur von Gewebe, z.B. Blut, im Innern eines legenden Körpers K.

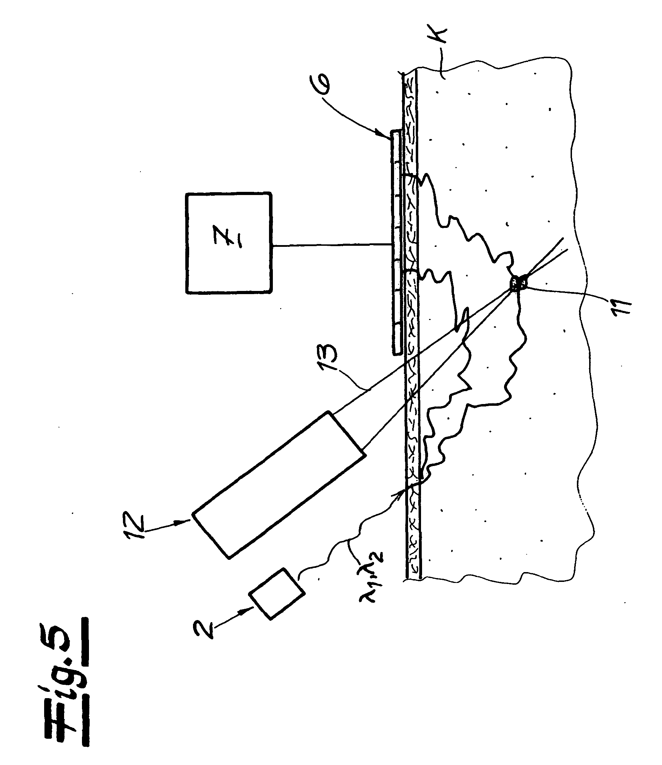

Dazu wird erfindungsgemäβ das Zielgebiet der Messung mit Hilfe von Ultraschallstrahlung markiert. Ein solches Verfahren ist in anderem Zusammenhang in der DE 103 11 408 B3 beschrieben worden. Die dort beschriebene Markierung eines Bereiches im Innern eines Körpers kann in entsprechender Weise auch zur Markierung eines Bereiches im Zuge der Temperaturmessung erfolgen. Dazu wird beispielhaft auf Fig. 5 verwiesen. Das Infrarot-Licht eines Lasers 2 wird in der beschriebenen Weise (für die Wellenlängen λ₁ und λ₂) in das Innere eines Körpers K eingestrahlt und es werden die rückgestreuten Photonen, welche die Absorption repräsentieren, mit einem Detektor 6 gemessen. Der Detektor 6 registriert nun nicht nur die im Bereich des Blutgefäßes 11 rückgestreuten Photonen, sondern auch eine Vielzahl weiterer Photonen, welche in anderen Bereichen gestreut wurden. Eine Markierung bzw. Selektion gelingt nun durch Einstrahlung von Ultraschallstrahlung 13 mit der in Fig. 5 dargestellten Ultraschall-Strahlungsquelle 12. Diese wird auf das Zielgebiet, nämlich das Blutgefäß 11 fokussiert. Dabei kann man sich, z.B. bei fließendem Blut - z.B. den Dopplereffekt zu Nutze machen, so wie dieses in der DE 103 11 408 B3 beschrieben ist. Die Ultraschallstrahlungsquelle 12 erzeugt gepulste Ultraschallstrahlung mit fester Pulslänge und fester Repetitionszeit. Über die Auswerteeinheit kann dann unter Berücksichtigung dieses Pulsverhaltens der Lichtanteil aus dem Detektor 6 extrahiert werden, welcher tatsächlich zu dem Volumen des Ultraschallfokus beiträgt. Einzelheiten sind in der DE 103 11 408 B3 und der DE 10 2006 036 920 beschrieben, welche sich jedoch nicht mit der Temperaturbestimmung, sondern mit der nichtinvasiven Messung der Konzentration von Blutbestandteilen befassen. Mit einer solchen nichtinvasiven Messung der Konzentration von Blutbestandteilen kann das erfindungsgemäße Verfahren dann im Übrigen auch kombiniert werden. Es gelingt folglich die nichtinvasive Messung der Konzentration von Blutbestandteilen, z.B. die Messung des Zuckergehaltes, wobei gleichzeitig auch eine Temperaturbestimmung erfolgen kann.

Da der durch den Ultraschall markierte Lichtanteil in der Praxis eventuell nicht nur von der Temperatur des beobachteten Ortes abhängt, sondern in gewissem Maß auch von dem Gradient der Temperatur der Oberfläche und des zu beobachteten Ortes abhängen kann, kann es zweckmäßig sein, zuvor eine Referenzmessung an der Oberfläche des Messkörpers, z.B. an der Haut vorzunehmen, wobei auch dort eine Markierung mittels Ultraschallfokus zweckmäßig sein kann. Die dort vorgenommene Messung hängt ausschließlich von der dortigen Temperatur und nicht von der Temperatur eventueller Zwischenlagen oder einem Temperaturgradienten ab, so dass anschließend eine Temperatumessung in der gewünschten Tiefe des Körpers stattfinden kann und dabei eine Temperatur-Differenzmessung erfolgt.

Schließlich kann es ergänzend zur Korrektur zweckmäßig sein, eine Korrekturmessung mit Hilfe einer isosbestischen Wellenlänge vorzunehmen. Einzelheiten sind dazu in den Figuren nicht dargestellt. Eine solche isosbestische Wellenlänge zeichnet sich dadurch aus, dass der rückgestreute Photonenstrom nur durch Streueffekte in den Zwischenlagen sowie in dem beobachteten Ort beeinflusst wird und von dem (optischen) Absorptionsvermögen des Wassers vollkommen unabhängig ist. Das Streuverhalten kann folglich aus der vorgenommenen Messung "heraus korrigiert" werden. Diese Referenz- und Korrekturmessungen können in der Praxis in unmittelbarem (zeitlichen) Zusammenhang mit der durchgeführten Temperaturmessung erfolgen und sofort in die Auswertung einfließen, so dass sich eine Vorrichtung zur Durchführung des erfindungsgemäßen Verfahrens gleichsam von selbst kalibriert.

## Patentansprüche

1. Verfahren zur nichtinvasiven, optischen Bestimmung der Temperatur von fließendem Blut im Innern eines Körpers,
wobei das zu untersuchende Blut mit infrarotem und/oder sichtbarem Licht im Bereich einer Absorptionslinie beleuchtet wird, deren Lage von der Temperatur des Bluts abhängt, und wobei die Absorption des Lichtes im Bereich der Absorptionslinie gemessen und aus dieser Messung durch Vergleich mit Kalibrierungsdaten die Temperatur ermittelt wird,
wobei das Blut mit zumindest zwei diskreten Lichtwellenlängen λ₁, λ₂ beleuchtet wird, welche im Bereich der Absorptionslinie auf unterschiedlichen Seiten des Absorptionsmaximums liegen,
wobei aus dem Verhältnis bzw. einem funktionellen Zusammenhang dieser beiden ermittelten Absorptionswerte A(λ₁), A(λ₂) zueinander zumindest ein von der Temperatur (T) abhängiger Messwert oder eine von der Temperatur abhängige Messfunktion bestimmt wird,
wobei aus diesem Messwert bzw. dieser Messfunktion durch Vergleich mit den zuvor aufgenommenen Kalibrierungsdaten die Temperatur bestimmt wird,
wobei zur Messung der Temperatur im Innern eines Körpers der Ort der Messung in der Blutbahn, mittels gepulster Ultraschallstrahlung markiert wird, indem die gepulste Ultraschallstrahlung auf die Blutbahn fokussiert wird und im Zuge der Messung der Absorption des Lichtes für die Temperaturmessung lediglich die Anteile des in den Detektor einfallenden Lichtes berücksichtigt werden, welche in zeitlicher Beziehung zu der Ultraschallstrahlung stehen.

2. Verfahren nach Anspruch 1, wobei der Messwert durch Bildung der Differenz der beiden beidseitig des Maximums liegenden Absorptionswerte oder durch Bestimmung der Steigung einer durch die Messpunkte verlaufenden Geraden bestimmt wird.

3. Verfahren nach Anspruch 1 oder 2, wobei das Medium mit Infrarot-Licht und/oder sichtbarem Licht mit Wellenlänge λ₁, λ₂ zwischen 600 nm und 2500 nm, vorzugsweise 800 nm bis 1600 nm beleuchtet wird.

4. Verfahren nach Anspruch 3, wobei die Messung im Bereich der Wasser-absorptionslinie um 970 nm erfolgt, wobei vorzugsweise einerseits Licht einer ersten Wellenlänge λ₁ zwischen 950 und 970 nm und andererseits Licht einer zweiten Wellenlänge λ₂ zwischen 975 und 1000 nm verwendet wird.

5. Verfahren nach Anspruch 3, wobei die Messung im Bereich der Wasser-absorptionslinie um 1450 nm durchgeführt wird.

6. Verfahren nach einem der Ansprüche 1 bis 5, wobei zur Bestimmung der Temperatur im Innern eines Körpers zunächst eine Referenzmessung der Temperatur an der Oberfläche des Körpers durchgeführt wird und wobei anschließend die Messung der Temperatur an einem Ort im Innern des Körpers durchgeführt wird.

7. Verfahren nach einem der Ansprüche 1 bis 6, wobei zur Bestimmung der Temperatur im Innern eines Körpers eine Korrekturmessung mit einer oder mehreren isosbestischen Wellenlängen durchgeführt wird, wobei das Medium bzw. der Körper mit Licht mit einer isosbestischen Wellenlänge beleuchtet wird, bei welcher der rückgestreute Lichtanteil ausschließlich von Streueffekten im Innern des Körpers und nicht von dem Absorptionsverhalten des Mediums, z.B. Wasser, abhängt.

## Claims

1. A method for the non-invasive, optical determination of the temperature of flowing blood inside a body,
wherein the blood which is to be examined is illuminated with infrared and/or visible light in the region of an absorption line, the position of which depends on the temperature of the blood, and wherein the absorption of the light in the region of the absorption line is measured and from this measurement the temperature is determined by comparison with calibration data,
wherein the blood is illuminated with at least two discrete light wavelengths λ₁, λ₂ which lie in the region of the absorption line on different sides of the absorption maximum,
wherein at least one measured value dependent on the temperature (T) or a measuring function dependent on the temperature is determined from the ratio or respectively a functional relation of these two determined absorption values A(λ₁), A(λ₂) to one another,
wherein the temperature is determined from this measured value or respectively this measuring function by comparison with the previously recorded calibration data,
wherein for measuring the temperature inside a body, the site of the measurement in the bloodstream is marked by means of pulsed ultrasonic radiation, by the pulsed ultrasonic radiation being focussed on the bloodstream and in the course of measurement of the absorption of the light for the temperature measurement only the portions of the incident light into the detector are taken into consideration, which are in time relationship to the ultrasonic radiation.

2. The method according to Claim 1, wherein the measured value is determined by forming the difference of the two absorption values lying on both sides of the maximum or by determining the inclination of a straight line running through the measuring points.

3. The method according to Claim 1 or 2, wherein the medium is illuminated with infrared light and/or visible light with wavelength λ₁, λ₂ between 600 nm and 2500 nm, preferably 800 nm to 1600 nm.

4. The method according to Claim 3, wherein the measurement takes place in the region of the water absorption line at about 970 nm, wherein preferably on the one hand light of a first wavelength λ₁ between 950 and 970 nm is used, and on the other hand light of a second wavelength λ₂ between 975 and 1000 nm is used.

5. The method according to Claim 3, wherein the measurement is carried out in the region of the water absorption line at about 1450 nm.

6. The method according to one of Claims 1 to 5, wherein for determining the temperature inside a body firstly a reference measurement of the temperature on the surface of the body is carried out and wherein subsequently the measurement of the temperature is carried out at a site inside the body.

7. The method according to one of Claims 1 to 6, wherein for determining the temperature inside a body a correction measurement with one or more isosbestic wavelengths is carried out, wherein the medium or respectively the body is illuminated with light with an isosbestic wavelength, in which the backscattered light portion is dependent exclusively on scatter effects in the interior of the body and not on the absorption behaviour of the medium, e.g. water.

## Revendications

1. Procédé destiné à la détermination optique non-invasive de la température du sang circulant à l'intérieur d'un corps,
dans lequel le sang à examiner est éclairé avec une lumière à infrarouge et/ou visible, au niveau d'une ligne d'absorption dont la position dépend de la température du sang, et dans lequel l'absorption de la lumière est mesurée au niveau de la ligne d'absorption et la température est calculée à partir de cette mesure par comparaison avec des données d'étalonnage,
dans lequel le sang est éclairé avec au moins deux longueurs d'onde lumineuse λ₁, λ₂ discrètes, lesquelles se situent sur différents côtés du maximum d'absorption au niveau de la ligne d'absorption,
sachant qu'à partir du rapport, respectivement, d'un rapport fonctionnel l'une par rapport à l'autre de ces deux valeurs d' absorption A(λ₁), A(λ₂) calculées, au moins une valeur de mesure dépendante de la température (T) ou une fonction de mesure dépendante de la température est déterminée,
sachant qu'à partir de cette valeur de mesure, respectivement de cette fonction de mesure, la température est déterminée par comparaison avec les données d'étalonnage enregistrées au préalable,
dans lequel pour mesurer la température à l'intérieur d'un corps, l'emplacement de la mesure dans la trajectoire du sang est marqué via un rayonnement aux ultra-sons pulsé, en ce que le rayonnement aux ultra-sons pulsé est mis au point sur la trajectoire du sang et suite à la mesure de l'absorption de la lumière pour la mesure de la température, seules les parties de lumière entrant dans le détecteur sont mesurées, lesquelles sont en rapport temporel avec le rayonnement aux ultra-sons.

2. Procédé selon la revendication 1, dans lequel la valeur de mesure est déterminée par formation de la différence des deux valeurs d'absorption se situant de chaque côté du maximum ou par détermination de l'inclinaison d'une droite passant à travers les points de mesure.

3. Procédé selon la revendication 1 ou 2, dans lequel le milieu est éclairé avec de la lumière à infrarouge et/ou visible présentant une longueur d'onde lumineuse λ₁, λ₂ entre 600nm et 2500 nm, de préférence de 800 nm à 1600 nm.

4. Procédé selon la revendication 3, dans lequel la mesure au niveau de la ligne d'absorption s'effectue à 970 nm, sachant que de préférence on emploie d'une part de la lumière d'une première longueur d'onde λ₁ entre 950 et 970 nm et d'autre part de la lumière d'une deuxième longueur d'onde λ₂ entre 975 et 1000 nm.

5. Procédé selon la revendication 3, dans lequel la mesure au niveau de la ligne d'absorption s'effectue à 1450 nm.

6. Procédé selon l'une des revendications 1 à 5, dans lequel pour déterminer la température à l'intérieur d'un corps, on effectue d'abord une mesure de référence de la température sur la surface du corps et dans lequel enfin, la mesure de la température est effectuée sur un emplacement à l'intérieur du corps.

7. Procédé selon l'une des revendications 1 à 6, dans lequel pour déterminer la température à l'intérieur d'un corps, on effectue une mesure de correction avec une ou plusieurs longueur(s) d'onde isobestique(s), sachant que le milieu, respectivement, le corps est éclairé avec de la lumière présentant une longueur d'onde isobestique, dans laquelle la part de lumière rétrodiffusée dépend exclusivement de phénomènes de diffusion à l'intérieur du corps et non du comportement d'absorption du milieu, par exemple de l'eau.
